# EUROPEAN PATENT SPECIFICATION

(11) **EP 3 741 433 B1**
(45) Date of publication and mention of the grant of the patent: **02.09.2026**
(21) Application number: 20185448.6
(22) Date of filing: 13.06.2013
(51) Int. Cl.: A61Q 19/00, A61K 8/34, A61K 8/891, A61K 8/06, A61K 8/892, A61K 8/894

(54) **WATER-RELEASING COSMETIC COMPOSITION**
WASSERFREISETZENDE KOSMETIKZUSAMMENSETZUNG
COMPOSITION COSMÉTIQUE LIBÉRANT DE L'EAU

(30) Priority: 21.06.2012 US 201213529059; 02.04.2013 US 201313855495
(43) Date of publication of application: 25.11.2020
(62) Document number(s) of the earlier application(s) in accordance with Art. 76 EPC: 13730783.1 / 2 864 000
(73) Proprietor: L'OREAL, 75008 Paris (FR)
(72) Inventor: CHIOU, Catherine, Saddle Brook, NJ New Jersey 07663 (US)
(74) Representative: Prinz & Partner mbB

(56) References cited:
- WO-A1-2010/059466
- US-A1- 2007 128 137
- US-A1- 2007 196 291
- US-A1- 2008 038 360
- US-A1- 2008 299 058
- US-A1- 2010 234 474
- US-A1- 2011 305 649
- "KSG SERIES 15-16-18-41-42-43-44-210-310-320-330-340-710-830-840", PRODUCT BROCHURE SHINETSU, XX, XX, 1 January 2004 (2004-01-01), pages 1,7,10, XP002424955

## Description

### FIELD OF THE INVENTION

The present disclosure is directed to cosmetic compositions and methods of using and producing cosmetic compositions. More specifically, the present disclosure is directed to a water-releasing cosmetic composition in the form of an emulsion having an aqueous phase including a hydrating agent and an oil phase including dimethicone and an emulsifying crosslinked siloxane elastomer. The water-releasing cosmetic composition converts from an emulsion to a plurality of droplets upon rubbing.

### BACKGROUND OF THE INVENTION

For various reasons associated in particular with greater comfort of use (softness, emollience and the like), current cosmetic compositions are usually in the form of an emulsion of the oil-in-water (O/W) type consisting of an aqueous-dispersing-continuous phase and an oily-dispersed-discontinuous phase, or of an emulsion of the water-in-oil (W/O) type consisting of an oily-dispersing-continuous phase and an aqueous-dispersed-discontinuous phase. O/W emulsions are usually preferred in the cosmetics field, because O/W emulsions comprise an aqueous phase as external phase, which gives the emulsions, when applied to the skin, a fresher, less greasy, less tacky, and lighter feel than W/O emulsions.

Many compositions, especially cosmetic compositions, have been developed for easy and comfortable application onto a targeted substrate. Unfortunately, many of these compositions are in fact difficult to apply and do not possess a smooth feel upon application. Moreover, compositions often have a tendency to feel tacky, yielding poor application and spreadability characteristics.

US 2007/0128137 A1 shows water-in-oil emulsion compositions comprising from about 0.1 % to about 15 % of a non-emulsifying crosslinked siloxane elastomer, from about 0.1 % to about 15 % of an emulsifying crosslinked elastomer and from about 40 % of a solvent for the non-emulsifying elastomer. The aqueous phase is present in an amount of from about 50 % to about 99 %. All examples use a mixture of hydrating agents comprising niacinamide.

US 2010/0234474 A1 discloses a water-in-oil HIPE (high internal phase emulsion) comprising water, an emulsifier, a steric stabilizer, a depletion stabilizer, oil, a non-emulsifying elastomer and a water-soluble active. The compositions shown are meant to enable an even application of the water-soluble active on skin.

In US 2011/0305649 A1 high internal phase emulsions are described which comprise an internal phase and an external phase. The internal phase is an aqueous phase, preferably comprising from about 40 % to about 95 % of a humectant, e.g. glycerin. The emulsion further comprises an emulsifier, a steric stabilizer, an oil, a non-emulsifying elastomer, and/or a depletion stabilizer.

US 2008/0038360 A1 shows personal care compositions comprising from about 0.1 % to about 15 % of an emulsifying silicone elastomer, from about 1 % to about 90 % of an aqueous phase and - in the oil phase - from about 0.001 % to about 5 % of a coated particulate comprising a core material and a coating material. As core material, e.g. mica, iron oxide, titanium dioxide, boron nitride, interference pigments and mixtures thereof can be used.

US 2008/0299058 A1 discloses a cosmetic composition comprising two individual formulations in a 1:9 to 9:1 ratio. The first formulation comprises from about 0.1 % to about 15 % of an emulsifying crosslinked siloxane elastomer, from about 1 % to about 50 % of a solvent for the emulsifying crosslinked siloxane elastomer and from about 40 % to about 99 % of an aqueous phase. The second formulation is recited as being different from the first formulation.

In US 2007/0196291 A1 cosmetic compositions including silicone polymers are presented. The silicone polymers have two different repeating units, wherein a first repeating unit has an organopolysiloxane residue and a second repeating unit has phosphate and amino functionality.

WO 20101059466 A1 discloses cosmetic compositions including silicone polymers with an emulsifying crosslinked siloxane elastomer, a noncrosslinked silicone emulsifier having a polyglycerin unit, a solvent for the emulsifying crosslinked siloxane elastomer, an amphiphilic active and water.

Although glycerin is a fairly low cost humectant or hydrating agent, problems arise when incorporating high levels of glycerin in cosmetic compositions. Incorporating high levels of glycerin, generally greater than 5%, results in a cosmetic compositions having a tacky and sticky feel upon application to skin. The tacky and sticky feel is undesirable to consumers. Several approaches, such as using light emollients, powders, or combinations thereof may reduce tackiness; however, the resulting cosmetic compositions may not provide sufficient consumer appeal and may still have residual tackiness that can be felt on the skin after application.

Therefore, it is desirable to provide a composition possessing a high level of glycerin without having a tacky feel and that is pleasing to consumers.

A cosmetic composition and methods of using and producing cosmetic compositions that do not suffer from one or more of the above drawbacks would be desirable in the art.

### BRIEF DESCRIPTION OF THE INVENTION

The object of the invention is solved by a water-releasing cosmetic composition according to claim 1, a method for preparing said water-releasing cosmetic composition according to claim 7 and the use of said water-releasing cosmetic composition according to claim 8.

According to the invention, a water-releasing cosmetic composition in the form of an emulsion, the composition consists of:
a) an aqueous phase consisting of a hydrating agent at a concentration, by weight, of 10 % to 50 %, based upon weight of the composition, wherein the hydrating agent is a hydrating agent selected from the group consisting of glycerin, PEG/PPG/polybutylene glycol-8/5/3 glycerin, butylene glycol, propylene glycol, dipropylene glycol, pentylene glycol, sorbitol, hexylene glycol, hydroxyethyl urea, sodium 5-oxo-2-pyrrolidinecarboxylate and mixtures thereof, or
   a hydrating agent selected from the group consisting of glycerin, dipropylene glycol or mixtures thereof, or a mixture of glycerin and/or of dipropylene glycol and of one or more other polyols selected from the group consisting of butylene glycol, propylene glycol, isoprene glycol, hexylene glycol, polyethylene glycols, sorbitol, sugars, methylpropanediol and 1,3-propanediol and mixtures thereof; water, and, optionally, a preservative system consisting of sodium benzoate and potassium sorbate,
b) an oil phase consisting of:
   dimethicone, at a concentration, by weight of 5 % to 20 %, based upon weight of the composition; and
   an emulsifying crosslinked siloxane elastomer at a concentration, by weight, of 0.5 % to 6 %, based upon weight of the composition; and
   optionally, a co-emulsifier at a concentration, by weight, of 0.05 % to 0.9 %, based upon weight of the composition, wherein the co-emulsifier is selected from polyether linear or branched polysiloxane copolymers; and
c) optionally, one or more of
   a cosmetically acceptable additive or adjuvant selected from the group of moisturizers, colorants, fillers, antioxidants, fragrances, dyes, neutralizing agents, pH-adjusting agents, conditioning agents, softening agents, botanical extracts or mixtures thereof, wherein the cosmetically acceptable additive or adjuvant may be soluble or dispersible in the aqueous phase and/or the oil phase;
   a cosmetic or dermatologic active agent selected from the group of free-radical scavengers, keratolytic agents, vitamins, anti-elastase agents, anti-collagenase agents, peptides, steroids, trace elements, extracts of algae, extracts of planktons, enzymes, coenzymes, flavonoids, ceramides, hydroxyl acids or mixtures thereof, wherein the cosmetic or dermatologic active agent may be soluble or dispersible in the aqueous phase and/or the oil phase; and
   a cosmetic powder;

wherein a phase ratio of the total weight of aqueous phase to the total weight of the oil phase is 5.0 to 12.0, wherein the phase ratio excludes any additional optional powders that may be added to the composition; and
wherein the cosmetic composition converts from an emulsion to a plurality of droplets upon rubbing, wherein the rubbing is carried out by placing 0.2 g of the composition on the back of a hand, applying the composition by circling with the middle finger and ring finger of the other hand and observing the water-releasing effect when the circling application reaches 20 cycles, wherein at least 10 to 20 bead-like water drops having an average diameter of 1 mm appear.

Other features and advantages of the present disclosure will be apparent from the following more detailed description of the preferred embodiments which illustrate, by way of example, the principles of the disclosure.

### BRIEF DESCRIPTION OF THE DRAWINGS

FIG. 1 is a microscopic photograph of a water-in-oil emulsion of the present disclosure including an internal water phase dispersed into a continuous oil phase.
FIG. 2 is a microscopic photograph of a water-in-oil emulsion of the present disclosure including an internal water phase dispersed into a continuous oil phase.
FIG. 3 is a microscopic photograph of a water-in-oil emulsion of the present disclosure including an internal aqueous phase dispersed into a continuous oil phase.

Wherever possible, the same reference numbers will be used throughout the drawings to represent the same parts.

### DETAILED DESCRIPTION OF THE INVENTION

"Keratinous tissue," as used herein, includes but is not limited to skin, hair, and nails.

"Homogenous" means substantially uniform throughout, i.e., a single phase mixture.

In the present application the term "ambient temperature" means a temperature of about 25° C.

In the present application the term "water-releasing," as used herein, describes the phenomenon wherein, upon application of a cosmetic composition, the shearing forces generated by the rubbing in or application of the cosmetic composition cause the water-in-oil type emulsion to rupture, thereby causing the internal aqueous phase to emerge in the form of droplets.

It has been surprisingly discovered by the inventor that high levels of hydrating agents, such as glycerin can be formed into a water-in-oil type emulsion in the form of a cream that has a transformative water-releasing effect upon rubbing into keratinous tissue. The transformative water-releasing effect is that the cream transforms into droplets containing the aqueous phase upon rubbing the cream into keratinous tissue. It has also been surprisingly discovered by the inventor that a composition containing greater than about 80% by weight of an aqueous phase forms a stable emulsion in the form of a cream. It has also been surprisingly discovered by the inventor that the cream provides a unique and refreshing sensory experience without the tackiness associated with incorporating high levels of hydrating agents like glycerin into cosmetic compositions. It has also been surprisingly discovered by the inventor that a stable water-in-oil emulsion cream is formed using only emulsifying crosslinked siloxane elastomers.

One advantage of an embodiment of the present disclosure includes providing a cosmetic composition for incorporating relatively high levels of aqueous based moisturizing ingredients (e.g. glycerin). Another advantage of an embodiment of the present disclosure includes providing cosmetic compositions that provide improved skin-feel properties. Yet another advantage of an embodiment of the present disclosure is providing a cosmetic composition having an aqueous phase at a concentration, by weight, of greater than about 80% based upon weight of the composition. Yet another advantage of an embodiment of the present disclosure is providing a keratinous tissue treatment composition that has stability against phase separation even under freeze/thaw cycling. Another advantage of an embodiment of the present disclosure includes a keratinous tissue treatment composition that achieves a smooth non-draggy rub-in upon initial application to the keratinous tissue.

The water-in-oil emulsion system of the present water-releasing cosmetic composition has a white, glossy cream appearance, or it may change to a transparent gel-like or matte appearance by a method of adjusting the refractive index, as known by those skilled in the art. When the cosmetic composition is applied to the skin in a conventional way, the cosmetic composition quickly releases bead-like droplets containing the aqueous phase, bringing about a novel and soothing feeling to consumers

### Aqueous Phase

The aqueous phase present in the cosmetic composition according to the disclosure includes glycerin, water, and other aqueous phase ingredients. The aqueous phase of the water-releasing cosmetic composition is at a concentration, by weight, of 50% to 90%, or alternatively 85% to 90% based upon weight of the cosmetic composition. In one embodiment, the aqueous phase may include at least one hydrating agent, water and optionally a preservative system.

### Hydrating Agent

The aqueous phase present in the cosmetic composition according to the disclosure includes a hydrating agent at a concentration, by weight, of 10% to 50% or alternatively about 10% to about 30% based upon weight of the composition.

The hydrating agents is selected from glycerol (glycerin), butylene glycol, propylene glycol, dipropylene glycol, hexylene glycol, sorbitol, PEG/PPG/polybutylene glycol-8/5/3 glycerin having 8 mol EO, 5 mol PO, 3 mol BO average, pentylene glycol, hydroxyethyl urea, sodium 5-oxo-2-pyrrolidinecarboxylate (sodium PCA) and mixtures thereof. According to some embodiment of the invention, the polyol chosen is glycerol (glycerin), dipropylene glycol or mixtures thereof, or a mixture of glycerol and/or of dipropylene glycol and of one or more other polyols selected from those indicated here: butylene glycol, propylene glycol, isoprene glycol, hexylene glycol, polyethylene glycols, sorbitol, sugars, methylpropanediol and 1,3-propanediol and mixtures thereof. A particularly suitable polyol for use with the present disclosure is glycerin.

In one embodiment, glycerin is incorporated in the cosmetic composition at levels greater than 5% or alternatively, greater than 10%, by weight, of the cosmetic composition or alternatively between about 10% to about 45%, by weight, of the cosmetic composition.

### Water

The aqueous phase present in the cosmetic composition according to the disclosure includes water at a concentration, by weight, of about 20% to about 85%, or alternatively about 35% to about 80% or alternatively about 40% to about 70%, based upon weight of the composition. The water used may be sterile demineralized water and/or a floral water such as rose water, cornflower water, camomile water or lime water, and/or a natural thermal or mineral water such as, for example: water from Vittel, water from the Vichy basin, water from Uriage, water from La Roche Posay, water from La Bourboule, water from Enghien-les-Bains, water from Saint Gervais-les-Bains, water from Neris-les-Bains, water from Allevar-les-Bains, water from Digne, water from Maizieres, water from Neyrac-les-Bains, water from Lons-le-Saunier, water from Eaux Bonnes, water from Rochefort, water from Saint Christau, water from Les Fumades, water from Tercis-les-Bains or water from Avene. The water phase may also comprise reconstituted thermal water, that is to say a water comprising trace elements such as zinc, copper, magnesium, etc., reconstituting the characteristics of a thermal water.

### Preservative System

The aqueous phase present in the cosmetic composition according to the disclosure includes a preservative system at a concentration, by weight, of about 0.1% to about 3%, or alternatively about 0.5% to about 2.5% or alternatively about 1% to about 2.0%, based upon weight of the composition. In a preferred embodiment, the preservative system includes an organic acid preservative system. A suitable example of a preservative system is sodium benzoate and potassium sorbate.

### Oil Phase

The oil phase present in the cosmetic composition according to the disclosure consists of dimethicone and an emulsifying crosslinked siloxane elastomer, and optionally a co-emulsifier. The oil phase of the water-releasing cosmetic composition is at a concentration, by weight, of about 5% to about 40%, or alternatively about 8% to about 30%, or alternatively about 10% to about 20%, based upon weight of the cosmetic composition.

### Dimethicone

The oil phase present in the cosmetic composition according to the disclosure includes dimethicone at a concentration, by weight, of 5% to 20%, or alternatively 7% to 15%, based upon weight of the composition.

### Emulsifying Crosslinked Siloxane Elastomer

The oil phase present in the cosmetic composition according to the disclosure includes an emulsifying crosslinked siloxane elastomer at a concentration, by weight, of 0.5% to 6%, or alternatively 1% to 5%, based upon weight of the composition.

Examples of suitable emulsifying crosslinked siloxane elastomers, include, but are not limited to, substituted or unsubstituted dimethicone/copolyol crosspolymer, dimethicone and dimethicone/PEG-10/15 crosspolymers, substituted or unsubstituted dimethicone/polyglyceryl crosspolymer, dimethicone and dimethicone/polyglycerin-3 crosspolymer. Such suitable emulsifying crosslinked siloxane elastomers are sold or made, for example, under the names of "KSG-210" a polyether-modified cross polymer with an INCI name of dimethicone (and) dimethicone/PEG-10/15 crosspolymer, and "KSG-710" a polyglycerin-modified crosspolymer with an INCI name of dimethicone (and) dimethicone/polyglycerin-3 crosspolymer, both available from ShinEtsu Silicones of America, Inc. (Akron, OH).

The water-releasing cosmetic composition of the present disclosure may also contain cosmetically acceptable additives or adjuvants as well as cosmetic or dermatologic active agents. Representative additives and adjuvants include, for example, water-soluble or water-miscible solvents or co-solvents, dispersion enhancing agents, moisturizers, colorants, fillers, antioxidants (e.g., EDTA, BHT, tocopherol), essential oils, fragrances, dyes, neutralizing or pH-adjusting agents (e.g., citric acid, triethylamine (TEA) and sodium hydroxide), conditioning or softening agents (e.g., panthenol and allantoin) and extracts such as botanical extracts. Additives and adjuvants may be present in the compositions in amounts generally ranging from about 0.01% to about 10% by weight. Examples of cosmetic active agents or dermatological active agents include sunscreen agents (e.g., inorganic sunscreen agent, such as titanium dioxide and zinc oxide and organic sunscreen agents, such as octocrylene, ethylhexyl methoxycinnamate, and avobenzone), free-radical scavengers, keratolytic agents, vitamins (e.g., Vitamin E and derivatives thereof), anti-elastase and anti-collagenase agents, peptides, fatty acid derivatives, steroids, trace elements, extracts of algae and of planktons, enzymes and coenzymes, flavonoids and ceramides, hydroxy acids and mixtures thereof, and enhancing agents. These ingredients may be soluble or dispersible in any water phase(s) or oil phase(s) that is/are present in the cosmetic composition (i.e., aqueous and/or fatty (oil) phase).

### Co-emulsifier

The oil phase present in the cosmetic composition according to the disclosure may optionally include a co-emulsifier at a concentration, by weight, of 0.05% to 0.9%, or alternatively 0.1% to 0.8%, based upon weight of the composition. If the co-emulsifier concentration exceeds 1% by weight of the cosmetic composition, then the cosmetic composition may still form an emulsion but the desirable transformative effect of cream changing to droplets upon rubbing is lost.

Suitable examples of co-emulsifiers include polyether substituted linear or branched polysiloxane copolymers. One preferred co-emulsifier is PEG-10 dimethicone available under the tradename of ES-5612 from Dow Corning Corporation (Midland, Michigan), or KF-6017 from Shin-Etsu (Akron, Ohio). Another preferred co-emulsifier is dimethicone (and) PEG/PPG-18/18 dimethicone available under the tradename of ES-5226 DM from Dow Corning Corporation (Midland, Michigan). Other suitable co-emulsifiers include, PEG-9 polydimethylsiloxyethyl dimethicone (and) PEG-9 available under the tradename KF-6028, and lauryl PEG-9 polydimethylsiloxyethyl dimethicone available under the tradename KF-6038, both available from Shin-Etsu (Akron, Ohio). Another suitable example of a co-emulsifier is polyoxyalkylene copolymers also known as silicone polyethers. Polyoxyalkylene copolymers are described in detail in U.S. Pat. 4,268,499, which is incorporated herein by reference in its entirety. A particularly preferred polyoxyalkylene copolymer is known by its CTFA designation as dimethicones copolyol. A particularly preferred form of dimethicone copolyol is supplied by Dow Corning as DC5225C.

### Optional Powders

The cosmetic composition of the present disclosure may optionally include cosmetic powders. The optional cosmetic powders provide formulas that are smoother and softer on the skin. Representative cosmetic powders include, but are not limited to talc, mica, magnesium carbonate, calcium carbonate, magnesium silicate, aluminum magnesium silicate, silica, titanium dioxide, zinc oxide, red iron oxide, yellow iron oxide, black iron oxide, polyethylene powder, methacrylate powder, polystyrene powder, silk powder, crystalline cellulose, starch, titanated mica, iron oxide titanated mica, bismuth oxychloride, and the like. Additional powders include, but are not limited to, inorganic powders such as gums, chalk, Fuller's earth, kaolin, sericite, muscovite, phlogopite, synthetic mica, lepidolite, biotite, lithia mica, vermiculite, aluminum silicate, starch, smectite clays, alkyl and/or trialkyl aryl ammonium smectites, chemically modified magnesium aluminum silicate, organically modified montmorillonite clay, hydrated aluminum silicate, fumed aluminum starch octenyl succinate barium silicate, calcium silicate, magnesium silicate, strontium silicate, metal tungstate, magnesium, silica alumina, zeolite, barium sulfate, calcined calcium sulfate (calcined gypsum), calcium phosphate, fluorine apatite, hydroxyapatite, ceramic powder, metallic soap (zinc stearate, magnesium stearate, zinc myristate, calcium palmitate, and aluminum stearate), colloidal silicone dioxide, and boron nitride; organic powder such as polyamide resin powder (nylon powder), cyclodextrin, methyl polymethacrylate powder, copolymer powder of styrene and acrylic acid, benzoguanamine resin powder, poly(ethylene tetrafluoride) powder, and carboxyvinyl polymer, cellulose powder such as hydroxyethyl cellulose and sodium carboxymethyl cellulose, ethylene glycol monostearate; inorganic white pigments such as magnesium oxide. A representative cosmetic powder includes, for example, polymethylsilsesquioxane. Cosmetic powders may be present in the compositions in amounts generally ranging from about 0.1% to about 5% by weight of the composition.

### Phase Ratio

The phase ratio is calculated by dividing the total weight of the aqueous phase by the total weight of the oil phase. The cosmetic composition of the present disclosure as a water-in-oil emulsion has a ratio of the aqueous phase to oil phase of 5.0 to 12.0, or alternatively, 5.0 to 10.0, or alternatively 7.0 to 9.0. The phase ratio excludes any additional optional powders that may be added to the composition.

### Hydration Index

The cosmetic composition of the present disclosure has a hydration index of greater than about 1.34.

The hydration index is calculated using the following equation ${IP}_{1 h} = \frac{Average \left[{\left({T}_{1 h}-{T}_{0}\right)}_{formula}-{\left({T}_{1 h}-{T}_{0}\right)}_{bare skin}\right]}{Average \left[{\left({T}_{1 h}-{T}_{0}\right)}_{reference}-{\left.{T}_{1 h}-{T}_{0}\right)}_{bare skin}\right]}$ where IP1h is the hydration index of the skin; T1h is the Corneometer reading one hour after applying the formula or reference, T0 is the initial Corneometer reading right after applying the formula or reference, (T1h-T0)formula is the difference between the Corneometer reading one hour after applying the formula of the present disclosure to the skin and the Corneometer reading right after applying the formula of the present disclosure to the skin; (T1h-T0)bare skin is the difference between the Corneometer reading at the one hour mark and initial reading of bare skin; (T1h-T0)reference is the difference between the Corneometer reading one hour after applying the reference cream (containing 7% glycerin) to the skin and Corneometer reading right after applying the reference cream (containing 7% glycerin) to the skin. The Corneometer readings were taken at ambient temperatures. The Corneometer used to measure the hydration index was Corneometer^{®} CM825, available from Courage + Khazaka, Köln, Germany.

### Process

The method for preparing the water-releasing cosmetic composition of the present disclosure, according to one embodiment, includes creating a stable water-in-oil emulsion with or without heating. In one embodiment, the process uses a cold-processing method which keeps the temperature below 30°C and more preferably at ambient temperature during emulsification. In an alternative embodiment, the process includes heating the water and oil phases to an elevated temperature which includes temperatures above 30°C to form the emulsion. The process includes mixing a first phase (aqueous) including a hydrating agent (e.g. glycerin), water, the preservative system and other ingredients. In one embodiment, the pH of the aqueous phase is adjusted using suitable and well-known pH adjusters to create optimal conditions to prevent any growth of undesired microbiological organisms. The process includes mixing a second phase (oil) including dimethicone and an emulsifying crosslinked siloxane elastomer. The process includes very slowly adding the first phase (aqueous) to the second phase (oil) while mixing and as viscosity of the mixture increases, mixing speed is increased to about 1200 rpm. After the first phase (aqueous) is mixed into the second phase (oil) a white, glossy, trembling cream emulsion is formed, where the cream releases droplets upon rubbing the cream into the skin.

### Viscosity

Viscosity is measured using a Brookfield Viscometer, using spindle T-D and speed set at 10 rpm. Viscosity of the present disclosure is about 40,000 cp to about 75,000 cp (1 cp being equal to 0,0001 Pa.s).

### Water-Releasing Effect

With respect to the present invention, a good water-releasing effect of the water-in-oil emulsion means that the water-releasing effect has an evaluation result of more than or equal to a score of 3 in the evaluation system described below. The test method and evaluation score of the test system are described below.

About 0.2 g of a water-in-oil emulsion sample of cosmetic composition is taken and placed on the back of a hand, then it is applied thereon by circling gently with the middle finger and ring finger of the other hand, and then the phenomenon of the water-releasing effect is observed when the circling application reaches 20 cycles, and evaluated by a 5-level scoring system. A score of 5 represents that more than 10 bead-like water drops having an average diameter of more than or equal to 3 mm appear, or more than 20 bead-like water drops having an average diameter of more than or equal to 1 mm appear. A score of 4 represents that 2-10 bead-like water drops having an average diameter of more than or equal to 3 mm appear, or 10-20 bead-like water drops having an average diameter of more than or equal to 1 mm appear and the bead-like water drops having an average of more than or equal to 3 mm are no more than 10. A score of 3 represents that 2-9 bead-like water drops having an average diameter of more than or equal to 1 mm appear and there is at most 1 bead-like water drop having an average diameter of more than or equal to 3 mm, or 10-20 bead-like water drops having an average diameter of 1 mm appear. A score of 2 represents that 2-9 bead-like water drops having an average diameter of 1 mm appear. A score of 1 represents that no water drop appears. Each level between scores 5 to 4, 4 to 3, 3 to 2, and 2 to 1 shows that the water-releasing effect is between the two end values described above, and the lower the score, the poorer the water-releasing effect.

In one embodiment, the water-releasing effect of the cosmetic composition of the present disclosure is about 4 to 5. In embodiments, having higher levels of glycerin, namely greater than 30%, the water-releasing effect of the cosmetic composition of the present disclosure is about 2 to 3.

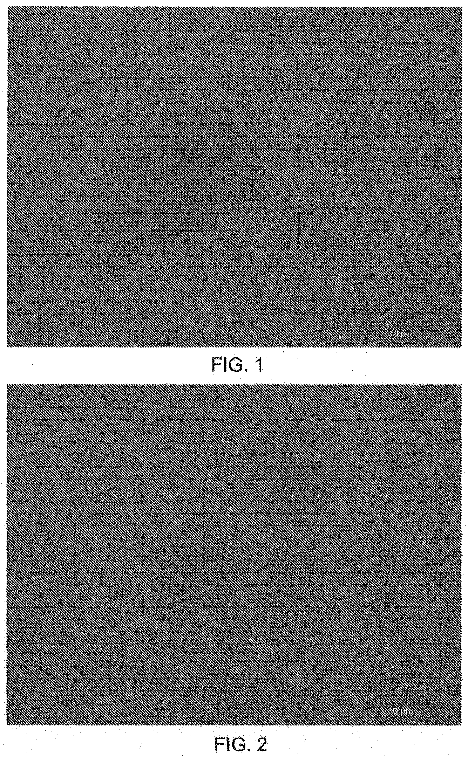

The water-silicone boundary of the water-in-oil emulsion of the present disclosure is stable as shown in FIGS. 1-3. The water-in-oil emulsion of the present disclosure includes an external or oil (silicone) phase surrounding non-uniform and larger droplet sizes of the internal aqueous phase. The non-uniform aqueous droplets range in size from approximately 0.1 microns to about 100 microns in diameter, with many aqueous drops having a diameter of about 50 microns.

### Tackiness Test

The tackiness of the cosmetic compositions is measured using a TA.XT Plus Texture Analyzer. The cosmetic composition is evenly applied on a Leneta drawdown card in the amount of 0.1g over an area of 2 cm x 6.5 cm and allowed to air-dry for 2 min. The drawdown card with cosmetic composition or test product is mounted on a testing platform. The measurement is made using a tack probe and is recorded with Exponent 32 software. The average of six (6) measurements for each formula is used in the analysis. The tackiness of the test product is expressed as the force (in Newtons) required to lift the tack probe from the test product surface. The higher the force required to remove the tack probe from the test product surface, the tackier the cosmetic composition. Generally, tackiness levels of greater than 20 Newtons will register a "tackiness" feeling with consumers. Tackiness levels of less than 20 Newtons are generally not considered to be "tacky" by consumers.

Upon application of the water-releasing cosmetic composition to keratinous tissue, the aqueous phase droplets are released from the emulsion and form droplets on the surface of a keratinous tissue as a result of the shearing forces used to apply the cosmetic composition to the keratinous tissue.

A method for treating keratinous tissue includes applying to the keratinous tissue the cosmetic composition of the present disclosure. The cosmetic composition of the present disclosure is in any desirable cosmetic form, such as, but not limited to, liquid lotions, creams, and mousses, can be applied to keratinous tissue to provide greater hydration.

The following examples are intended to further illustrate the present invention. Unless otherwise indicated, all parts are by weight.

### EXAMPLES

**Table 1**

| **Phase** | **INCI Name** | **Ex. 1 (inventive)** | **Ex. 2 (inventive)** | **Ex. 3 (comparative)** |
|---|---|---|---|---|
| A | Dimethicone/P EG 10/15 Crosspolymer | 1.2 | 1.2 | 1.2 |
| A | Dimethicone, 5 cst | 13.8 | 13.8 | 13.8 |
| B1 | Glycerin | 15 | 45 | 0 |
| B1 | Propanediol | 3 | 3 | 3 |
| B1 | Sodium Benzoate (preservative) | 0.2 | 0.2 | -- |
| B1 | Potassium Sorbate (preservative) | 0.1 | 0.1 | -- |
| B1 | Sodium Citrate | 0.2 | 0.2 | 0.2 |
| | Preservative | -- | -- | 0.7 |
| B1 | Sodium Chloride | 0.5 | 0.5 | 0.5 |
| B1 | Water | 65.9 | 35.9 | 80.5 |
| B2 | Citric Acid | 0.1 | 0.1 | 0.1 |
| | Total (wt/wt%): | 100 | 100 | 100 |
| | Texture | White, trembling gel-like cream. Water droplets released upon rubbing. | Translucent, white gel-like cream. Water droplets released upon rubbing | Frosty white cream. Water droplets released upon rubbing |
| | Microscope | W/Si, boundary ok, large water droplet size. | W/Si, boundary ok, water droplets are smaller and more uniform than Example 1. | W/Si, boundary ok, large water droplet size. |
| | Viscosity (cp or mPa·s) | 74,000 | 63,000 | 60,000 |
| | Tackiness (Newtons) | 7.81 | 10.19 | 8.99 |
| | Water-Releasing Effect | 4 to 5 | 2 to 3 | 4 to 5 |

**Table 2**

| **Phase** | **INCI Name** | **Ex. 4 (comparative)** |
|---|---|---|
| A | Polyglyceryl-4 Diisostearate/Polyhydroxystearate/Sebacate | 3 |
| A | Ethylhexyl Palmitate | 7 |
| A | Caprylic/Capric Triglyceride | 4 |
| A | Octyldodecanol | 4 |
| A | Pentaerythrityl Tetraethylhexanoate | 1 |
| B | Water | QS |
| B | Glycerin | 10 |
| B | Sodium Chloride | 2 |
| B | Preservatives | 0.7 |
| | Total (wt/wt%): | 100 |
| | Viscosity (cp or mPa·s) | 14,200 |
| | Tackiness (Newtons) | 29.63 |
| | Water-Releasing Effect | 1 |

The method of making each of the examples provided in Tables 1 and 2 is generally the same. The examples in Table 1 include inventive examples and comparative examples having a water-releasing effect. The example in Table 2 is a comparative example illustrating the tackiness of glycerin in a typical water-in-oil emulsion.

In making each of the examples in Tables 1 and 2, the following procedure is used. The ingredients of Phase B (aqueous) are mixed together in a side kettle at ambient temperature or higher temperatures. The ingredients of Phase A (oil phase) are mixed together in a main kettle at ambient temperature or higher temperatures. The mixture of Phase B ingredients (aqueous phase) is slowly added to the mixture of Phase A ingredients (oil phase), while mixing. As viscosity of the mixture increases, mixing speed in the main kettle is increased to about 1200 rpm. After the first phase (aqueous) is mixed into the second phase (oil) a water-in-oil emulsion is formed.

### Example 1

The water-in-oil emulsion of inventive Example 1 is prepared according to the procedure outlined above and includes adding a pH adjuster, citric acid, to the aqueous phase prior to mixing Phase B (aqueous phase) with Phase A (oil phase). Example 1 includes 15% glycerin. The emulsion formed in Example 1 is a white, trembling gel-like cream that releases droplets upon rubbing. The water/silicone emulsion boundary layer is stable and includes droplets having various droplet sizes, with some droplets as large as 50 microns or greater (see FIGS 1 and 2). The viscosity of Example 1 is measured using a Brookfield Viscometer, using spindle T-D and speed set at 10 rpm. The viscosity of Example 1 is about 74,000 cp (mPa.s). The tackiness of Example 1 is measured using a TA.XT Plus Texture Analyzer. The tackiness of Example 1 is 7.81 Newtons, which indicates little or no tackiness feeling when applied to the skin. The water-releasing effect of Example 1 is measured by placing about 0.2 g of the cosmetic composition on the back of a hand, then applying thereon by circling gently with the middle finger and ring finger of the other hand. The phenomenon of the water-releasing effect is observed when the circling application reaches 20 cycles. Approximately 5-7 bead-like droplets of more than or equal to 3 mm appears and approximately 15-18 bead-like droplets having an average diameter of more than or equal to 1 mm appear. The water-releasing effect of the water-in-oil emulsion of Example 1 is about 4 to 5. The hydration index of Example 1 is measured to be about 1.59.

### Example 2

The water-in-oil emulsion of inventive Example 2 is prepared according to the procedure outlined above. Example 2 includes 45% glycerin. The emulsion formed in Example 2 is translucent, white gel-like cream that releases droplets upon rubbing. The water/silicone emulsion boundary layer is stable with smaller and more uniform water droplets. The viscosity of Example 2 is measured using a Brookfield Viscometer, using spindle T-D and speed set at 10 rpm. The viscosity of Example 2 is 63,000 cp (mPa.s). The tackiness of Example 2 is measured using a TA.XT Plus Texture Analyzer as described above. The tackiness of Example 2 is 10.19 Newtons, which indicates there is little or no tackiness feeling when applied to the skin. The water-releasing effect of Example 2 is measured by placing about 0.2 g of the cosmetic composition on the back of a hand. The cosmetic composition is then applied thereon by circling gently with the middle finger and ring finger of the other hand. The phenomenon of the water-releasing effect is observed when the circling application reaches about 20 cycles. Approximately 9-11 bead-like droplets having an average diameter of 1 mm appear. The water-releasing effect of the water-in-oil emulsion of Example 2 is about 2 to 3. Example 2 incorporates very high levels of glycerin and does not have a tacky feel.

### Example 3

The water-in-oil emulsion of Example 3 is a comparative example and is prepared according to the procedure outlined above. Example 3 does not include glycerin. The emulsion formed in Example 3 is a frosty-looking, white cream that releases droplets upon rubbing. The water/silicone emulsion boundary layer is stable with various droplet sizes. The viscosity of Example 3 is measured using a Brookfield Viscometer, using spindle T-D and speed set at 10 rpm. The viscosity of Example 3 is 60,000 cp (mPa.s). The tackiness of Example 3 is measured using a TA.XT Plus Texture Analyzer. The tackiness of Example 3 is 8.99 Newtons. Example 3 has little or no tackiness feeling when applied to the skin. The water-releasing effect of Example 3 is measured by placing about 0.2 g of the cosmetic composition on the back of a hand. The cosmetic composition is then applied thereon by circling gently with the middle finger and ring finger of the other hand. The phenomenon of the water-releasing effect is observed when the circling application reaches 20 cycles. Approximately 5-7 bead-like droplets of more than or equal to 3 mm appear and approximately 15-18 bead-like droplets having an average diameter of more than or equal to 1 mm appear. The water-releasing effect of the water-in-oil emulsion of Example 3 is about 4 to 5. Example 3 does not include glycerin; therefore, Example 3 does not provide the hydrating and moisturizing properties of inventive Examples 1 and 2.

### Example 4

The water-in-oil emulsion of Example 4 is a comparative example and is prepared according to the procedure outlined above. The water-in-oil emulsion of Example 4 is a typical water-in-oil emulsion. Example 4 includes about 10% by weight glycerin. The emulsion formed in Example 4 is a glossy, white cream that does not release droplets upon rubbing. The water/oil emulsion boundary layer is stable and includes uniform droplets evenly dispersed within the emulsion. The viscosity of Example 4 is measured using a Brookfield Viscometer, using spindle T-D and speed set at 10 rpm. The viscosity of Example 4 is 14,200 cp (mPa.s). The tackiness of Example 4 is measured using a TA.XT Plus Texture Analyzer for a tackiness of 29.63 Newtons. The tackiness of Example 4 is greater than 20 Newtons, as such, Example 4 feels tacky when applied to the skin. The water-releasing effect of Example 4 is measured by placing about 0.2 g of the cosmetic composition on the back of a hand. The cosmetic composition is applied thereon by circling gently with the middle finger and ring finger of the other hand. The phenomenon of the water-releasing effect is observed when the circling application reaches 20 cycles. No bead-like droplets having an average diameter of more than or equal to 1 mm appeared. The water-releasing effect of the water-in-oil emulsion of Example 4 is about 1; therefore, Example 4 has no water-releasing effect.

**Table 3**

| **Phase** | **INCI Name** | **Ex. 5** | **Ex. 6** | **Ex. 7** | **Ex. 8** |
|---|---|---|---|---|---|
| A | Dimethicone/P EG-10/15 Crosspolvmer | 1.2 | 1.2 | 1.7 | 1.2 |
| A | PEG-10 Dimethicone | 0.07 | 0 | 0 | 0 |
| A | Dimethicone | 10.8 | 10.8 | 10.3 | 10.8 |
| B1 | Water | 64.96 | 66.78 | 38.28 | 70.28 |
| B1 | Glycerin | 15 | 15 | 45 | 15 |
| B1 | Propanediol | 5 | 3 | 3 | 3 |
| B1 | Preservatives | QS | QS | QS | QS |
| B1 | Disodium EDTA | 0.1 | 0.1 | 0.1 | 0.1 |
| B1 | Sodium Citrate | 0.2 | 0.2 | 0.2 | 0.2 |
| B1 | Sodium Chloride | 0.5 | 0.5 | 0.5 | 0.5 |
| B2 | Citric Acid | 0.12 | 0.12 | 0.12 | 0.12 |
| C | Polymethylsilsesquioxane | 1 | 0.5 | 0 | 0 |
| | Total (wt/wt%): | 100 | 100 | 100 | 100 |
| | Brookfield Viscosity (mPa.s): | 45,000 | 40,000 | 50,000 | 56,000 |
| | Total Oil Phase (%) | 12.07 | 12 | 12 | 10 |
| | Total Water Phase (%) | 86.93 | 87.5 | 88 | 90 |
| | Ratio (water phase / oil phase)* | 7.20 | 7.29 | 7.33 | 9.00 |
| | Texture | White, glossy trembling cream. Water droplets released upon rubbing | White, glossy trembling cream. Water droplets released upon rubbing | Translucent, gel-like trembling cream. Water droplets released upon rubbing | White, glossy trembling cream. Water droplets released upon rubbing |
| | Microscope | W/Si, good boundary | W/Si, good boundary | W/Si, good boundary | W/Si, good boundary |
| | Tackiness (Newtons) | 7.70 | 7.52 | 12.3 | 6.48 |
| | Water Releasing Effect | 4 to 5 | 4 to 5 | 2 to 3 | 5 |

| | | | | | |
|---|---|---|---|---|---|
| • Excludes powder (Phase C) | | | | | |

In making each of the examples in Tables 3, the following procedure is used.

The ingredients of Phase B1 (aqueous) are mixed together in a side beaker with a rotor/stator mixer until all solids are dissolved, giving a clear solution. If needed, Phase B1 (aqueous) can be gently heated to about 40-45°C until all solids are dissolved. The final pH of the aqueous phase is adjusted to about 5.0 ±0.3 with Phase B2 (citric acid). The ingredients of Phase A (oil phase) are placed in a main beaker and are mixed well with a propeller mixer at about 600-700 RPM and set aside. The mixture of aqueous phase ingredients (Phase B1 and B2 combined) are slowly added to the mixed ingredients of Phase A (oil phase) using a prop mixer over a period of 10-15 minutes for an about 1kg batch. As the viscosity of the mixture increases, the stirring speed is increased from 700 rpm to about 1200 rpm. As the aqueous phase is mixed into the oil phase a water-in-oil emulsion is formed. Optionally, powders are added to the batch and are mixed into the water-in-oil emulsion.

### Example 5

The water-in-oil emulsion of inventive Example 5 is prepared according to the procedure outlined above. Example 5 includes 15% glycerin. The total weight percentage of the aqueous or water phase is about 86.93 and the total weight percent of the oil phase is about 12.07, making the a ratio of the aqueous phase to oil phase about 7.2. The emulsion formed in Example 5 is a white, glossy, trembling cream that releases droplets upon rubbing. The water/silicone emulsion boundary layer is stable and includes droplets having various droplet sizes, with some droplets as large as 50 microns or greater. The viscosity of Example 5 is measured using a Brookfield Viscometer, using spindle T-D and speed set at 10 rpm for 1 minute. The viscosity of Example 5 is about 45,000 cp (mPa.s). The tackiness of Example 5 is measured using a TA.XT Plus Texture Analyzer. The tackiness of Example 5 is 7.70 Newtons, which indicates little or no tackiness feeling when applied to the skin. The water-releasing effect of Example 5 is measured by placing about 0.2 g of the cosmetic composition on the back of a hand, then applying thereon by circling gently with the middle finger and ring finger of the other hand. The phenomenon of the water-releasing effect is observed when the circling application reaches 20 cycles. Approximately 5-7 bead-like droplets of more than or equal to 3 mm appears and approximately 15-20 bead-like droplets having an average diameter of more than or equal to 1 mm appear. The water-releasing effect of the water-in-oil emulsion of Example 5 is about 4 to 5.

### Example 6

The water-in-oil emulsion of inventive Example 6 is prepared according to the procedure outlined above. Example 6 includes 15% glycerin. The total weight percentage of the aqueous or water phase is about 87.5 and the total weight percent of the oil phase is about 12, making the a ratio of the aqueous phase to oil phase about 7.29. The emulsion formed in Example 6 is a white, glossy, trembling cream that releases droplets upon rubbing. The water/silicone emulsion boundary layer is stable and includes droplets having various droplet sizes, with some droplets as large as 50 microns or greater. The viscosity of Example 6 is measured using a Brookfield Viscometer, using spindle T-D and speed set at 10 rpm for 1 minute. The viscosity of Example 6 is about 40,000 cp (mPa.s). The tackiness of Example 6 is measured using a TA.XT Plus Texture Analyzer. The tackiness of Example 6 is 7.52 Newtons, which indicates little or no tackiness feeling when applied to the skin. The water-releasing effect of Example 6 is measured by placing about 0.2 g of the cosmetic composition on the back of a hand, then applying thereon by circling gently with the middle finger and ring finger of the other hand. The phenomenon of the water-releasing effect is observed when the circling application reaches 20 cycles. Approximately 7-10 bead-like droplets of more than or equal to 3 mm appears and approximately 15-20 bead-like droplets having an average diameter of more than or equal to 1 mm appear. The water-releasing effect of the water-in-oil emulsion of Example 6 is about 4 to 5.

### Example 7

The water-in-oil emulsion of inventive Example 7 is prepared according to the procedure outlined above. Example 7 includes 45% glycerin. The total weight percentage of the aqueous or water phase is about 88 and the total weight percent of the oil phase is about 12, making the a ratio of the aqueous phase to oil phase about 7.33. The emulsion formed in Example 7 is a white, trembling cream that releases droplets upon rubbing. The water/silicone emulsion boundary layer is stable and includes droplets having various droplet sizes, with some droplets as large as 50 microns or greater. The viscosity of Example 7 is measured using a Brookfield Viscometer, using spindle T-D and speed set at 10 rpm for 1 minute. The viscosity of Example 7 is about 50,000 cp (mPa.s). The tackiness of Example 7 is measured using a TA.XT Plus Texture Analyzer. The tackiness of Example 7 is 12.30 Newtons, which indicates little or no tackiness feeling when applied to the skin. The water-releasing effect of Example 7 is measured by placing about 0.2 g of the cosmetic composition on the back of a hand, then applying thereon by circling gently with the middle finger and ring finger of the other hand. The phenomenon of the water-releasing effect is observed when the circling application reaches 20 cycles. Approximately 5-10 bead-like droplets having an average diameter of more than or equal to 1 mm appear. The water-releasing effect of the water-in-oil emulsion of Example 7 is about 2 to 3.

### Example 8

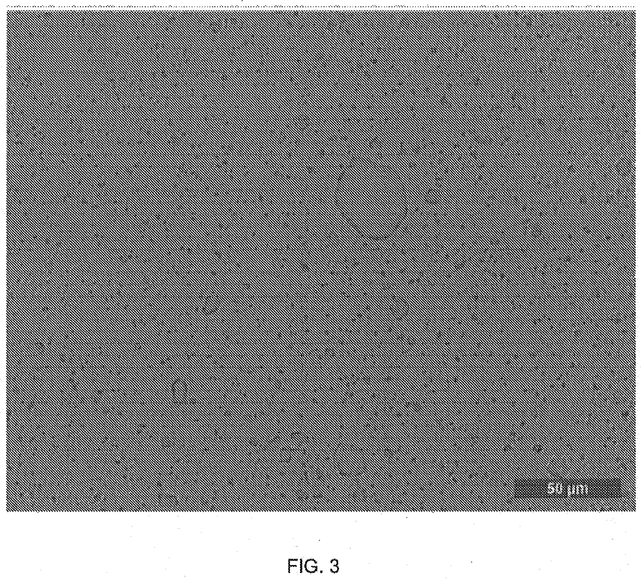

The water-in-oil emulsion of inventive Example 8 is prepared according to the procedure outlined above. Example 8 includes 15% glycerin. The total weight percentage of the aqueous or water phase is about 90 and the total weight percent of the oil phase is about 10, making the a ratio of the aqueous phase to oil phase about 9. The emulsion formed in Example 8 is a white, glossy, trembling cream that releases droplets upon rubbing. The water/silicone emulsion boundary layer is stable and includes droplets having various droplet sizes, with some droplets as large as 50 microns or greater (see FIG. 3). The viscosity of Example 8 is measured using a Brookfield Viscometer, using spindle T-D and speed set at 10 rpm for 1 minute. The viscosity of Example 8 is about 56,000 cp (mPa.s). The tackiness of Example 8 is measured using a TA.XT Plus Texture Analyzer. The tackiness of Example 8 is 6.48 Newtons, which indicates little or no tackiness feeling when applied to the skin. The water-releasing effect of Example 8 is measured by placing about 0.2 g of the cosmetic composition on the back of a hand, then applying thereon by circling gently with the middle finger and ring finger of the other hand. The phenomenon of the water-releasing effect is observed when the circling application reaches 20 cycles. Approximately 10-12 bead-like droplets of more than or equal to 3 mm appears and approximately 20-30 bead-like droplets having an average diameter of more than or equal to 1 mm appear. The water-releasing effect of the water-in-oil emulsion of Example 8 is about 5.

### Hydrating Efficacy Study

Hydrating Efficacy of the present disclosure was evaluated in a double-blind test. The formula provided in Example 5 was evaluated to determine the hydrating efficacy compared to a known positive control, L'Oreal HYDRA RENEWAL^{®} (having a glycerin content of about 7%).

Twenty-three (23) women with dry inner forearm skin tested Example 5 and positive control (L'Oreal HYDRA RENEWAL^{®}). The test was conducted over a four day period and the study conditions were as follows:

**Table 4 - Study Conditions**

| **Day** | **Internal Temp (F)** | **Internal RH** | **External Temp (F)** | **External RH** |
|---|---|---|---|---|
| 1 | 66.3 | 10.1 | 22.0 | 25.0 |
| 2 | 66.0 | 9.0 | 16.0 | 42.0 |
| 3 | 66.5 | 10.0 | 17.0 | 50.0 |
| 4 | 66.4 | 9.6 | 14.0 | 50.0 |

An average of three (3) measurements were taken at each site for data analysis. The measurements were taken with Corneometer^{®} CM825, available from Courage + Khazaka, Köln, Germany.

A paired t-test was used to compare the treated and untreated sites at baseline. To determine the treatment effect of the formulation of Example 5 and the positive control, differences from the baseline (T24 hrs - Baseline, T48 hrs - Baseline, and T72 hrs - Baseline) of the treated and untreated sites for each product were compared using a paired t-test. For the positive control, twenty-four (24) hour measurement was done on bare skin before reapplication of positive control on days 2 and 3 of the study. The positive control was reapplied every 24 hours during the study. The formulation provided in Example 5 was applied only one time at the start of the study on day 1. The results of Corneometer measurements and calculated p-Values for the study are below.

**Table 5 - Study Results**

| **Time (Hours)** | **Sample Size** | **Untreated Sites** | **Positive Control - Hydra Renewal** | **p-Value** | **Example 5** | **p-Value** |
|---|---|---|---|---|---|---|
| Baseline (Day 1) | 23 | 24.68 (± 5.09) | 24.15 (±4.11) | 0.504 | 23.97 (±4.98) | 0.308 |
| Diff - Twenty-Four (24) Hours Post Application (Day 2) | 23 | 0.28 (±4.04) | 2.30 (±3.80) | 0.011 | 3.07 (±2.59) | 0.002 |
| Diff - Forty-Eight (48) Hours Post Application (Day 3) | 23 | 0.03 (±2.45) | 2.28 (±2.89)* | 0.008 | 1.94 (±2.33) | 0.013 |
| Diff - Seventy-Two (72) Hours Post Application (Day 4) | 22 | -1.30 (±2.59) | 2.77 (±4.03)* | < 0.001 | 0.36 (±2.88) | 0.009 |

| | | | | | | |
|---|---|---|---|---|---|---|
| *Reapplied every 24 hours | | | | | | |

As expected, the study results show that there was no significant difference in hydration between the control sites, the positive control sites, or Example 5 treated sites at baseline on day 1. The significance level for the study was set at a p-Value of ≤0.05. The study shows that the positive control, HYDRA RENEWAL^{®}, statistically hydrated the skin twenty-four (24) hours after product application on each previous day, when compared to the untreated control. The study also shows that formulation of Example 5 statistically hydrated the skin at twenty-four (24), forty-eight(48), and seventy-two (72) hours after product application, when compared to the untreated control. This study indicates that formulation of Example 5 provides a 72-hour period of extended hydrating benefit on the skin.

**Table 6**

| **Phase** | **INCI Name** | **Ex. 9** | **Ex. 10** | **Ex. 11** |
|---|---|---|---|---|
| A | Dimethicone/PEG-10/15 Crosspolymer | - | 0.72 | 1.2 |
| A | Dimethicone/Polyglycerin-3 Crosspolymer | 1.2 | 0.96 | - |
| A | PEG-9 Polydimethylsiloxyethyl Dimethicone (and) PEG-9 | 0.07 | - | - |
| A | Lauryl PEG-9 Polydimethylsiloxyethyl Dimethicone | - | 0.07 | - |
| A | Dimethicone (and) PEG/PPG-18/18 Dimethicone | - | - | 0.07 |
| A | Dimethicone (and) Dimethiconol | - | - | 1 |
| A | Dimethicone | 10.8 | 10.32 | 10.8 |
| B1 | Water | 20 | 20 | 20 |
| B1 | p-Anisic Acid | 0.15 | 0.15 | 0.15 |
| B1 | Sodium Hydroxide (50% soln) | 0.05 | 0.05 | 0.05 |
| B2 | Water | 46.98 | 48.13 | 37.13 |
| B2 | PEG/PPG/Polybutylene Glycol -8/5/3 Glycerin | 10 | - | - |
| B2 | Butylene Glycol | 3 | - | - |
| B2 | Dipropylene Glycol | 3 | - | - |
| B2 | Pentylene Glycol | - | 5 | - |
| B2 | Hexylene Glycol | - | 5 | - |
| B2 | Propylene Glycol | - | 5 | - |
| B2 | Sorbitol | - | - | 5 |
| B2 | Hydroxyethyl Urea | - | - | 15 |
| B2 | Sodium PCA | - | - | 5 |
| B2 | Propanediol | 3 | 3 | 3 |
| B2 | Phenoxyethanol | 0.5 | 0.3 | 0.3 |
| B2 | Chlorphenesin | 0.3 | 0.3 | 0.3 |
| B2 | Disodium EDTA | 0.1 | 0.1 | 0.1 |
| B2 | Sodium Citrate | 0.2 | 0.2 | 0.2 |
| B2 | Sodium Chloride | 0.5 | 0.5 | 0.5 |
| B3 | Citric Acid | 0.15 | 0.2 | 0.2 |
| | Total (wt/wt%): | 100 | 100 | 100 |
| | Brookfield Viscosity (cp or mPa.s): | 48,000 | 60,000 | 53,000 |
| | Total Oil Phase (%) = | 12.07 | 12.07 | 13.07 |
| | Total Water Phase (%) = | 87.93 | 87.93 | 86.93 |
| | Ratio (water phase / oil phase) = | 7.29 | 7.29 | 6.65 |
| | Texture | White, glossy, trembling cream. Water droplets released upon rubbing. | White, glossy, trembling cream. Water droplets released upon rubbing. | White, glossy, trembling cream. Water droplets released upon rubbing. |
| | Microscope | W/Si, good boundary | W/Si, good boundary | W/Si, good boundary |
| | Water Releasing Effect | 5 | 5 | 5 |

In making each of the inventive examples in Tables 6, the following procedure is used.

The ingredients of Phase B1 (aqueous) are mixed together in a side beaker with a rotor/stator mixer until all solids are dissolved, giving a clear solution. The ingredients of Phase B2 (aqueous) are combined and mixed in a beaker. The mixed ingredients of Phase B1 are combined with the mixed ingredients of Phase B2 and mixed with a rotor/stator mixer and gently heated to about 40-45° until all solids are dissolved. The final pH of the aqueous phase is adjusted to about 5.0 ±0.3 with citric acid (Phase B3). The ingredients of Phase A (oil phase) are placed in a main beaker and are mixed well with a propeller mixer at about 600-700 RPM and set aside. The mixture of aqueous phase ingredients (Phase B1, B2 and B3 combined) are slowly added to the mixed ingredients of Phase A (oil phase) using a prop mixer over a period of 10-15 minutes for a 1 kg batch. As the viscosity of the mixture increases, the stirring speed is increased from 700 RPM to about 1200 RPM. As the aqueous phase is mixed into the oil phase, a water-in-oil emulsion is formed. Optionally, powders are added to the batch and are mixed into the water-in-oil emulsion.

### Example 9

The water-in-oil emulsion of inventive Example 9 is prepared according to the procedure outlined above. Example 9 includes a hydrating agent, by weight, of about 16%, based upon weight of the composition. The hydrating agent is a mixture, by weight, of about 10% PEG/PPG/Polybutylene glycol-8/5/3 glycerin, about 3% butylene glycol, and about 3% dipropylene glycol. The total weight percentage of the aqueous or water phase is about 87.93 and the total weight percent of the oil phase is about 12.07, making the ratio of the aqueous phase to oil phase about 7.29. The emulsion formed in Example 9 is a white, glossy, trembling cream that releases water droplets upon rubbing. The viscosity of Example 9 is measured using a Brookfield Viscometer, using spindle T-D and speed set at 10 rpm for 1 minute. The viscosity of Example 9 is about 48,000 cp (mPa·s). The water-releasing effect of Example 9 is measured by placing about 0.2 g of the cosmetic composition on the back of a hand, then applying thereon by circling gently with the middle finger and ring finger of the other hand. The phenomenon of the water-releasing effect is observed when the circling application reaches 20 cycles. Approximately 10-12 bead-like droplets of more than or equal to 3 mm appears and approximately 20-30 bead-like droplets having an average diameter of more than or equal to 1 mm appear. The water-releasing effect of the water-in-oil emulsion of Example 9 is about 5.

### Example 10

The water-in-oil emulsion of inventive Example 10 is prepared according to the procedure outlined above. Example 10 includes a hydrating agent, by weight, of about 15%, based upon weight of the composition. The hydrating agent is a mixture, by weight, of about 5% pentylene glycol, about 5% hexylene glycol, and about 5% propylene glycol. The total weight percentage of the aqueous or water phase is about 87.93 and the total weight percent of the oil phase is about 12.07, making the ratio of the aqueous phase to oil phase about 7.29. The emulsion formed in Example 10 is a white, glossy, trembling cream that releases water droplets upon rubbing. The viscosity of Example 10 is measured using a Brookfield Viscometer, using spindle T-D and speed set at 10 rpm for 1 minute. The viscosity of Example 10 is about 60,000 cp (mPa·s). The water-releasing effect of Example 10 is measured by placing about 0.2 g of the cosmetic composition on the back of a hand, then applying thereon by circling gently with the middle finger and ring finger of the other hand. The phenomenon of the water-releasing effect is observed when the circling application reaches 20 cycles. Approximately 10-12 bead-like droplets of more than or equal to 3 mm appears and approximately 20-30 bead-like droplets having an average diameter of more than or equal to 1 mm appear. The water-releasing effect of the water-in-oil emulsion of Example 10 is about 5.

### Example 11

The water-in-oil emulsion of inventive Example 11 is prepared according to the procedure outlined above. Example 11 includes a hydrating agent, by weight, of about 25%, based upon weight of the composition. The hydrating agent is a mixture, by weight, of about 5% sorbitol, about 15% hydroxyethyl urea, and about 5% sodium PCA. The total weight percentage of the aqueous or water phase is about 86.93 and the total weight percent of the oil phase is about 13.07, making the ratio of the aqueous phase to oil phase about 6.65. The emulsion formed in Example 11 is a white, glossy, trembling cream that releases water droplets upon rubbing. The viscosity of Example 11 is measured using a Brookfield Viscometer, using spindle T-D and speed set at 10 rpm for 1 minute. The viscosity of Example 11 is about 53,000 cp (mPa·s). The water-releasing effect of Example 11 is measured by placing about 0.2 g of the cosmetic composition on the back of a hand, then applying thereon by circling gently with the middle finger and ring finger of the other hand. The phenomenon of the water-releasing effect is observed when the circling application reaches 20 cycles. Approximately 10-12 bead-like droplets of more than or equal to 3 mm appears and approximately 20-30 bead-like droplets having an average diameter of more than or equal to 1 mm appear. The water-releasing effect of the water-in-oil emulsion of Example 11 is about 5.

**Table 7**

| **Phase** | **INCI Name** | **Ex. 12 (comparative)** |
|---|---|---|
| | Dimethicone(6 cst) (and) | |
| | Dimethicone/PEG-10/15 | |
| A | Crosspolymer (76/24) | 4 |
| A | Dimethicone (and) Dimethiconol (88/12) | 1 |
| A | Dimethicone, 6 cst | 6 |
| A | Trisiloxane, 1.0 cst | 16 |
| B | Water | 59.3 |
| B | Phenoxyethanol | 0.6 |
| B | Caprylyl glycol | 0.2 |
| B | Hexylene glycol | 0.1 |
| B | Iodopropynyl Butylcarbamate (10%) | 0.1 |
| B | Polyaminopropyl Biguanide (20% in water) | 0.2 |
| B | Butylene glycol | 2 |
| B | Glycerin | 10 |
| B | Sodium Citrate | 0.5 |
| | Total (wt/wt%): | 100 |
| | Brookfield Viscosity (cp or mPa.s): | 5000 |
| | Total Oil Phase (%) = | 27 |
| | Total Water Phase (%) = | 73 |
| | Ratio (water phase / oil phase) = | 2.7 |
| | Texture | Translucent, milky serum; watery on skin upon application. No "water-beading/releasing" effect. |
| | Microscope | Unstable. W/Si with leaking boarder, indicating potential instability of emulsion. |
| | Water Releasing Effect (scale of 1 to 5) = | 1 |

In making comparative Example 12, the following procedure was used. The ingredients of Phase B (aqueous) are mixed in together in a side breaker using a stirring bar to mix well and dissolve all solids. The ingredients of Phase A (oil phase) are placed in a main beaker and mixed well with a propeller mixer at about 600-700 RPM and set aside. The mixture of aqueous phase ingredients (Phase B) are slowly added to the mixed ingredients of Phase A using a prop mixer over a period of 10-15 minutes for a 1 kg batch. As viscosity slowly increased, the stirring speed is increased from 700 RPM to 1000 RPM to form a serum.

Comparative Example 12, in contrast to the present disclosure, has a total weight percentage of the aqueous phase or water phase of about 73% and a total weight percentage of oil of about 27%, making the ratio of the aqueous phase to oil phase about 2.7. Comparative Example 12 forms a translucent, milky serum that is watery on skin upon application. The viscosity of comparative Example 12 is measured using a Brookfield Viscometer, using spindle T-D and speed set at 10 rpm for 1 minute. The viscosity of comparative Example 12 is about 5,000 cp (mPa·s). The water-releasing effect of comparative Example 12 is measured by placing about 0.2g of the cosmetic composition on the back of a hand, then applying thereon by circling gently with the middle finger and ring finger of the other hand. No bead-like droplets having an average diameter of more than or equal to 1 mm appeared. The water-releasing effect of the serum of comparative Example 12 is about 1; therefore, comparative Example 12 has no water-releasing effect.

Comparative Example 12 is generally unstable. The microscope shows that the W/Si boundary has a leaking boarder, indicating potential instability of emulsion. Though the serum of comparative Example 12 initially forms as an emulsion, after 3 days of freeze-thaw cycles, the serum completely separates.

## Claims

1. A water-releasing cosmetic composition in the form of an emulsion, the composition consisting of:
a) an aqueous phase consisting of
a hydrating agent at a concentration, by weight, of 10 % to 50 %, based upon weight of the composition,
wherein the hydrating agent is
a hydrating agent selected from the group consisting of glycerin, PEG/PPG/polybutylene glycol-8/5/3 glycerin, butylene glycol, propylene glycol, dipropylene glycol, pentylene glycol, sorbitol, hexylene glycol, hydroxyethyl urea, sodium 5-oxo-2-pyrrolidinecarboxylate and mixtures thereof, or
a hydrating agent selected from the group consisting of glycerin, dipropylene glycol or mixtures thereof, or a mixture of glycerin and/or of dipropylene glycol and of one or more other polyols selected from the group consisting of butylene glycol, propylene glycol, isoprene glycol, hexylene glycol, polyethylene glycols, sorbitol, sugars, methylpropanediol and 1,3-propanediol and mixtures thereof,
water,
and, optionally, a preservative system consisting of sodium benzoate and potassium sorbate;
b) an oil phase consisting of:
dimethicone, at a concentration, by weight of 5 % to 20 %, based upon weight of the composition; and
an emulsifying crosslinked siloxane elastomer at a concentration, by weight, of 0.5 % to 6 %, based upon weight of the composition; and
optionally, a co-emulsifier at a concentration, by weight, of 0.05 % to 0.9 %, based upon weight of the composition, wherein the co-emulsifier is selected from polyether linear or branched polysiloxane copolymers; and
c) optionally, one or more of
a cosmetically acceptable additive or adjuvant selected from the group of moisturizers, colorants, fillers, antioxidants, fragrances, dyes, neutralizing agents, pH-adjusting agents, conditioning agents, softening agents, botanical extracts or mixtures thereof, wherein the cosmetically acceptable additive or adjuvant may be soluble or dispersible in the aqueous phase and/or the oil phase;
a cosmetic or dermatologic active agent selected from the group of free-radical scavengers, keratolytic agents, vitamins, anti-elastase agents, anti-collagenase agents, peptides, steroids, trace elements, extracts of algae, extracts of planktons, enzymes, coenzymes, flavonoids, ceramides, hydroxyl acids or mixtures thereof, wherein the cosmetic or dermatologic active agent may be soluble or dispersible in the aqueous phase and/or the oil phase; and
a cosmetic powder;
wherein a phase ratio of the total weight of aqueous phase to the total weight of the oil phase is 5.0 to 12.0, wherein the phase ratio excludes any additional optional powders that may be added to the composition; and
wherein the cosmetic composition converts from an emulsion to a plurality of droplets upon rubbing, wherein the rubbing is carried out by placing 0.2 g of the composition on the back of a hand, applying the composition by circling with the middle finger and ring finger of the other hand and observing the water-releasing effect when the circling application reaches 20 cycles, wherein at least 10 to 20 bead-like water drops having an average diameter of 1 mm appear.

2. The cosmetic composition of claim 1, wherein the emulsifying crosslinked siloxane elastomer comprises a substituted or unsubstituted dimethicone/copolyol crosspolymer, preferably wherein the emulsifying crosslinked siloxane elastomer is dimethicone/PEG-10/15 crosspolymer.

3. The cosmetic composition of claim 1, wherein the emulsifying crosslinked siloxane elastomer comprises a substituted or unsubstituted dimethicone/polyglyceryl crosspolymer, preferably wherein the emulsifying crosslinked siloxane elastomer is dimethicone/polyglycerin-3 crosspolymer.

4. The cosmetic composition of claim 1, wherein the co-emulsifier is a polyoxyalkylene copolymer, preferably wherein the co-emulsifier is selected from the group consisting of PEG-10 dimethicone, PEG-9 polydimethylsiloxyethyl dimethicone and PEG-9, lauryl PEG-9 polydimethylsiloxyethyl dimethicone, dimethicone and PEG/PPG-18/18 dimethicone, and combinations thereof.

5. The cosmetic composition of claim 1, wherein the phase ratio of the total weight of the aqueous phase to the oil phase is 5.0 to 10.0, preferably wherein the ratio of the aqueous phase to the oil phase is 7.0 to 9.0.

6. The cosmetic composition of claim 1, wherein the cosmetic powder is present at a concentration, by weight, of 0.1% to 5%, based upon weight of the composition.

7. A process for preparing the cosmetic composition according to claim 1, comprising:
mixing the aqueous phase;
mixing the oil phase; and
slowly adding the mixed aqueous phase to the mixed oil phase while mixing, forming an water-in-oil emulsion.

8. Use of a cosmetic composition according to claim 1, wherein the composition is applied to a keratinous substrate in a cosmetic treatment for moisturizing said substrate

9. The cosmetic composition of claim 1, wherein the aqueous phase is at a concentration, by weight, of 85 % to 90 %, based upon weight of the composition.

## Patentansprüche

1. Wasser freisetzende kosmetische Zusammensetzung in Form einer Emulsion, wobei die Zusammensetzung aus Folgendem besteht:
a) einer wässrigen Phase, bestehend aus
einem Hydratisierungsmittel in einer Konzentration von 10 Gew.-% bis 50 Gew.-%, bezogen auf das Gewicht der Zusammensetzung,
wobei das Hydratisierungsmittel
ein aus der aus Glycerin, PEG/PPG/Polybutylenglykol-8/5/3-Glycerin, Butylenglykol, Propylenglykol, Dipropylenglykol, Pentylenglykol, Sorbit, Hexylenglykol, Hydroxyethylharnstoff, Natrium-5-oxo-2-pyrrolidincarboxylat und Mischungen davon bestehenden Gruppe ausgewähltes Hydratisierungsmittel ist oder
ein aus der aus Glycerin, Dipropylenglykol oder Mischungen davon bestehenden Gruppe ausgewähltes Hydratisierungsmittel oder eine Mischung aus Glycerin und/oder aus Dipropylenglykol und einem oder mehreren anderen aus der aus Butylenglykol, Propylenglykol, Isoprenglykol, Hexylenglykol, Polyethylenglykolen, Sorbit, Zuckern, Methylpropandiol und 1,3-Propandiol und Mischungen davon bestehenden Gruppe ausgewählten Polyolen ist,
Wasser,
und optional einem aus Natriumbenzoat und Kaliumsorbat bestehenden Konservierungsstoffsystem,
b) einer Ölphase, bestehend aus:
Dimeticon in einer Konzentration von 5 Gew.-% bis 20 Gew.-% bezogen auf das Gewicht der Zusammensetzung und
einem emulgierenden vernetzten Siloxanelastomer in einer Konzentration von 0,5 Gew.-% bis 6 Gew.-% bezogen auf das Gewicht der Zusammensetzung und
optional einem Co-Emulgator in einer Konzentration von 0,05 Gew.-% bis 0,9 Gew.-% bezogen auf das Gewicht der Zusammensetzung, wobei der Co-Emulgator aus linearen oder verzweigten Polyether-Polysiloxan-Copolymeren ausgewählt ist, und
c) optional einem oder mehreren aus
einem kosmetisch verträglichen Zusatzstoff oder Hilfsstoff, der aus der Gruppe von Feuchtigkeitsspendern, Farbstoffen, Füllstoffen, Antioxidantien, Duftstoffen, Färbemitteln, Neutralisationsmitteln, pH-Einstellmitteln, Konditionierungsmitteln, Erweichungsmitteln, Pflanzenextrakten oder Mischungen davon ausgewählt ist, wobei der kosmetisch verträgliche Zusatzstoff oder Hilfsstoff in der wässrigen Phase und/oder der Ölphase löslich oder dispergierbar sein kann,
einem kosmetischen oder dermatologischen Wirkstoff, der aus der Gruppe von Radikalfängern, Keratolytika, Vitaminen, Anti-Elastase-Mitteln, Anti-Kollagenase-Mitteln, Peptiden, Steroiden, Spurenelementen, Extrakten aus Algen, Extrakten aus Plankton, Enzymen, Coenzymen, Flavonoiden, Ceramiden, Hydroxysäuren oder Mischungen davon ausgewählt ist, wobei der kosmetische oder dermatologische Wirkstoff in der wässrigen Phase und/oder der Ölphase löslich oder dispergierbar sein kann, und
einem kosmetischen Pulver,
wobei ein Phasenverhältnis des Gesamtgewichts der wässrigen Phase zum Gesamtgewicht der Ölphase 5,0 bis 12,0 beträgt, wobei das Phasenverhältnis etwaige zusätzliche optionale Pulver ausschließt, die der Zusammensetzung zugesetzt werden können, und
wobei sich die kosmetische Zusammensetzung beim Reiben von einer Emulsion in mehrere Tröpfchen umwandelt, wobei das Reiben durchgeführt wird, indem 0,2 g der Zusammensetzung auf den Handrücken gegeben werden, die Zusammensetzung durch Kreisen mit Mittelfinger und Ringfinger der anderen Hand aufgetragen wird und die Wasser freisetzende Wirkung beobachtet wird, wenn die Auftragung durch Kreisen 20 Zyklen erreicht, wobei mindestens 10 bis 20 perlenartige Wassertropfen mit einem mittleren Durchmesser von 1 mm erscheinen.

2. Kosmetische Zusammensetzung nach Anspruch 1, bei der das emulgierende vernetzte Siloxanelastomer ein substituiertes oder unsubstituiertes Dimeticon/Copolyol-Kreuzpolymer umfasst, vorzugsweise bei der das emulgierende vernetzte Siloxanelastomer Dimeticon/PEG-10/15-Kreuzpolymer ist.

3. Kosmetische Zusammensetzung nach Anspruch 1, bei der das emulgierende vernetzte Siloxanelastomer ein substituiertes oder unsubstituiertes Dimeticon/Polyglyceryl-Kreuzpolymer umfasst, vorzugsweise bei der das emulgierende vernetzte Siloxanelastomer Dimeticon/Polyglycerin-3-Kreuzpolymer ist.

4. Kosmetische Zusammensetzung nach Anspruch 1, bei der der Co-Emulgator ein Polyoxyalkylen-Copolymer ist, vorzugsweise bei der der Co-Emulgator aus der aus PEG-10-Dimeticon, PEG-9-Polydimethylsiloxyethyl-dimeticon und PEG-9, Lauryl-PEG-9-Polydimethylsiloxyethyldimeticon, Dimeticon und PEG/PPG-18/18-Dimeticon sowie Kombinationen daraus bestehenden Gruppe ausgewählt ist.

5. Kosmetische Zusammensetzung nach Anspruch 1, bei der das Phasenverhältnis des Gesamtgewichts der wässrigen Phase zur Ölphase 5,0 bis 10,0 beträgt, vorzugsweise bei der das Verhältnis der wässrigen Phase zur Ölphase 7,0 bis 9,0 beträgt.

6. Kosmetische Zusammensetzung nach Anspruch 1, bei der das kosmetische Pulver in einer Konzentration von 0,1 Gew.-% bis 5 Gew.-% bezogen auf das Gewicht der Zusammensetzung vorliegt.

7. Verfahren zur Herstellung der kosmetischen Zusammensetzung nach Anspruch 1, das Folgendes umfasst:
Mischen der wässrigen Phase,
Mischen der Ölphase und
langsames Zugeben der gemischten wässrigen Phase zu der gemischten Ölphase unter Mischen, wobei eine Wasser-in-Öl-Emulsion gebildet wird.

8. Verwendung einer kosmetischen Zusammensetzung nach Anspruch 1, bei der die Zusammensetzung bei einer kosmetischen Behandlung auf ein keratinöses Substrat aufgetragen wird, um dem Substrat Feuchtigkeit zu spenden.

9. Kosmetische Zusammensetzung nach Anspruch 1, bei der die wässrige Phase in einer Konzentration von 85 Gew.-% bis 90 Gew.-% bezogen auf das Gewicht der Zusammensetzung vorliegt.

## Revendications

1. Composition cosmétique libérant de l'eau sous forme d'émulsion, la composition étant composée :
a) d'une phase aqueuse composée
d'un agent hydratant à une concentration de 10% à 50% en poids par rapport au poids de la composition,
l'agent hydratant étant
un agent hydratant choisi dans le groupe constitué de glycérine, de PEG/PPG/polybutylène glycol-8/5/3-glycérine, de butylène glycol, de propylène glycol, de dipropylène glycol, de pentylène glycol, de sorbitol, d'hexylène glycol, d'hydroxyéthylurée, de 5-oxo-2-pyrrolidinecarboxylate de sodium et de leurs mélanges, ou
un agent hydratant choisi dans le groupe constitué de glycérine, de dipropylène glycol ou de leurs mélanges ou d'un mélange de glycérine et/ou de dipropylène glycol et d'un ou de plusieurs autres polyols choisis dans le groupe constitué de butylène glycol, de propylène glycol, d'isoprène glycol, d'hexylène glycol, des polyéthylène glycols, de sorbitol, de sucres, de méthyl propanediol et de propane-1,3-diol et de leurs mélanges,
d'eau,
et, facultativement, d'un système de conservation composé de benzoate de sodium et de sorbate de potassium ;
b) d'une phase huileuse composée :
de diméthicone à une concentration de 5% à 20% en poids par rapport au poids de la composition ; et
d'un élastomère siloxane réticulé émulsifiant à une concentration de 0,5% à 6% en poids par rapport au poids de la composition ; et
facultativement d'un co-émulsifiant à une concentration de 0,05% à 0,9% en poids par rapport au poids de la composition, le co-émulsifiant étant choisi parmi les copolymères polyéther-polysiloxane linéaires ou ramifiés ; et
c) facultativement d'un ou plusieurs parmi
un additif ou adjuvant cosmétiquement acceptable choisi dans le groupe des hydratants, des colorants, des agents de comblement, des antioxydants, des parfums, des agents colorants, des agents neutralisants, des agents d'ajustement du pH, des agents conditionneurs, des agents adoucissants, des extraits de plantes ou leurs mélanges, l'additif ou l'adjuvant cosmétiquement acceptable pouvant être soluble ou dispersible dans la phase aqueuse et/ou la phase huileuse ;
un agent actif cosmétique ou dermatologique choisi dans le groupe des piégeurs de radicaux libres, des agents kératolytiques, des vitamines, des agents anti-élastase, des agents anti-collagénase, des peptides, des stéroïdes, des oligoéléments, des extraits d'algues, des extraits de planctons, des enzymes, des coenzymes, des flavonoïdes, des céramides, et des acides hydroxyliques ou de leurs mélanges, l'agent actif cosmétique ou dermatologique pouvant être soluble ou dispersible dans la phase aqueuse et/ou la phase huileuse ; et
une poudre cosmétique ;
un rapport de phase entre le poids total de la phase aqueuse et le poids total de la phase huileuse étant de 5,0 à 12,0, le rapport de phase excluant toute poudre supplémentaire facultative qui peut être ajoutée à la composition ; et
la composition cosmétique se transformant d'une émulsion en une pluralité de gouttelettes lors d'un frottement, le frottement étant effectué en plaçant 0,2 g de la composition sur le dos de la main, en appliquant la composition en effectuant un mouvement de rotation avec le majeur et l'annulaire de l'autre main, et en observant l'effet de libération d'eau lorsque l'application par rotation atteint 20 cycles, au moins 10-20 gouttelettes d'eau en forme de perles présentant un diamètre moyen de 1 mm apparaissant.

2. Composition cosmétique selon la revendication 1, dans laquelle l'élastomère siloxane réticulé émulsifiant comprend un polymère réticulé diméthicone/copolyol substitué ou non substitué, de préférence dans laquelle l'élastomère siloxane réticulé émulsifiant est un polymère réticulé diméthicone/ PEG-10/15.

3. Composition cosmétique selon la revendication 1, dans laquelle l'élastomère siloxane réticulé émulsifiant comprend un polymère réticulé diméthicone/polyglycéryle substitué ou non substitué, de préférence dans laquelle l'élastomère siloxane réticulé émulsifiant est un polymère réticulé diméthicone/polyglycérine-3.

4. Composition cosmétique selon la revendication 1, dans laquelle le co-émulsifiant est un copolymère de polyoxyalkylène, de préférence dans laquelle le co-émulsifiant est choisi dans le groupe constitué de PEG-10 diméthicone, de PEG-9 polydiméthylsiloxyéthyldiméthicone et de PEG-9, de lauryl PEG-9 polydiméthylsiloxyéthyldiméthicone, de diméthicone et de PEG/PPG-18/18 diméthicone et de leurs combinaisons.

5. Composition cosmétique selon la revendication 1, dans laquelle le rapport de phases entre le poids total de la phase aqueuse et celui de la phase huileuse est de 5,0 à 10,0, de préférence dans laquelle le rapport entre la phase aqueuse et la phase huileuse est de 7,0 à 9,0.

6. Composition cosmétique selon la revendication 1, dans laquelle la poudre cosmétique est présente à une concentration de 0,1% à 5% en poids par rapport au poids de la composition.

7. Procédé de préparation de la composition cosmétique selon la revendication 1, comprenant :
le mélange de la phase aqueuse ;
le mélange de la phase huileuse ; et
l'ajout lent de la phase aqueuse mélangée à la phase huileuse mélangée tout en mélangeant, formant ainsi une émulsion eau dans huile.

8. Utilisation d'une composition cosmétique selon la revendication 1, dans laquelle la composition est appliquée sur un substrat kératinique lors d'un traitement cosmétique pour hydrater le substrat.

9. Composition cosmétique selon la revendication 1, dans laquelle la phase aqueuse est présente à une concentration de 85% à 90% en poids par rapport au poids de la composition.
